# EUROPÄISCHE PATENTSCHRIFT

(11) **EP 0 636 126 B1**
(45) Veröffentlichungstag und Bekanntmachung des Hinweises auf die Patenterteilung: **08.01.1997**
(21) Anmeldenummer: 93909365.4
(22) Anmeldetag: 08.04.1993
(51) Int. Cl.: C07D 307/92

(54) **VERFAHREN ZUR HERSTELLUNG VON SCLAREOLID**
PROCESS FOR PRODUCING SCLAREOLIDE
PROCEDE DE PRODUCTION DE SCLAREOLIDE

(30) Priorität: 16.04.1992 DE 4212731
(43) Veröffentlichungstag der Anmeldung: 01.02.1995
(73) Patentinhaber: Henkel Kommanditgesellschaft auf Aktien, 40191 Düsseldorf (DE)
(72) Erfinder: SCHNEIDER, Markus, D-4100 Duisburg 1 (DE); STALBERG, Theo, D-4019 Monheim (DE); GERKE, Thomas, D-4040 Neuss (DE)
(86) Internationale Anmeldenummer: EP9300874
(87) Internationale Veröffentlichungsnummer: WO9321174

(56) Entgegenhaltungen:
- DE-A- 3 942 358
- FR-A- 2 676 229
- US-A- 3 050 532

## Beschreibung

Die Erfindung betrifft ein Verfahren zur Herstellung von Sclareolid aus Sclareol und/oder Abienol.

Für die im folgenden verwendeten Trivialnamen sowie abkürzenden Stoffbezeichnungen in Verbindung mit der in runden Klammern nachgestellten Ziffer wird zur näheren Erläuterung auf den Beispielteil verwiesen, aus dem der zugehörige IUPAC-Name sowie die entsprechende Strukturformel entnommen werden können.

Ambroxan (7) ist ein wertvoller Ambrariechstoff, der in einer Stoffwechselausscheidung des Pottwals enthalten ist (vergleiche Ullmanns Enzyklopädie der technischen Chemie, Band 20, Seite 283, Weinheim 1981). Die zunehmende Nachfrage nach Ambroxan hat bei begrenzten natürlichen Ressourcen in den letzten Jahren zur Entwicklung von Verfahren geführt, durch die Ambroxan aus preiswerten Rohstoffen synthetisch zugänglich ist. Dabei hat sich insbesondere Sclareolid (6) als wichtige Ambroxan-Vorstufe herausgestellt, so daß das Interesse vieler Arbeitsgruppen der Entwicklung von Verfahren gegolten hat, durch die Sclareolid aus natürlichen Rohstoffen, insbesondere Sclareol (1), synthetisch zugänglich wird.

Gemäß US 30 50 532 läßt sich Sclareol zunächst mit Kaliumpermanganat unter alkalischen Reaktionsbedingungen zum Hydroxyketon (3) oxidieren, das ohne weitere Isolierung mit Eisessig in den Enolethern (4) umgewandelt wird. Dieser wird anschließend entweder mit Kaliumpermanganat oder Chromsäure oxidiert. Das erhaltene Oxidationsprodukt wird verseift und dann zum Sclareolid (6) cyclisiert. Dieses Verfahren hat jedoch den Nachteil, daß zur Oxidation mit Kaliumpermanganat gearbeitet wird, das ökologisch bedenklich bzw. giftig ist. Darüber hinaus läßt sich das daraus entstehende MnO₂ (Braunstein) bei der Aufarbeitung nur sehr mühsam abfiltrieren.

Aus der deutschen Patentanmeldung DE 39 42 358 ist schließlich ein Verfahren bekannt, bei dem Sclareol zunächst mit Hypochloritsalzen in Gegenwart von Rutheniumsalzen oxidativ zum Hydroxyketon (3) und/oder zum Enolether (4) abgebaut, das so erhaltene und isolierte Zwischenprodukt mit Persäuren und/oder Persäuresalzen oxidiert und nach Verseifung und saurem Ringschluß in Sclareolid überführt wird. Sclareolid ist nach diesem Verfahren in einer Ausbeute von ca. 65% - bezogen auf Sclareol - zugänglich.

Wegen der Schlüsselstellung des Ambroxans auf dem Gebiet der Riechstoffe besteht daher ein genereller Bedarf, verbesserte synthetische Zugangsmöglichkeiten zu entwickeln. Dieser Bedarf erstreckt sich insbesondere auf Verfahrensverbesserungen einzelner Teilschritte sowie die Entwicklung alternativer Zugangsmöglichkeiten zur Herstellung von wichtigen Zwischen-bzw. Vorprodukten des Ambroxans. Die Aufgabe der vorliegenden Erfindung bestand deshalb darin, ausgehend von Sclareol und strukturell nahe verwandten Verbindungen einen verbesserten Zugang zum Sclareolid zu eröffnen.

Diese Aufgabe wurde erfindungsgemäß dadurch gelöst, daß man Sclareol (1) und/oder Abienol (2) in wäßrigem Medium und in Abwesenheit eines organischen Lösungsmittels mit der 10,5- bis 25-fachen molaren Menge eines Oxidationsmittels in Gegenwart eines Ruthenium-Katalysators und eines Emulgators zunächst zu einem Rohprodukt umsetzt und dieses anschließend entweder in Gegenwart einer Base in das Salz von 8α-Hydroxy-11-carboxy-12,13,14,15,16-pentanorlabdan, im folgenden als Hydroxysäure (5) bezeichnet, überführt und dieses in saurem Medium weiter zu Sclareolid cyclisiert oder einer Nachreaktion bei erhöhter Temperatur unterwirft und anschließend destilliert, wobei die Nachreaktion gewünschtenfalls auch im Zuge der Destillation in situ erfolgen kann.

Gegenstand der vorliegenden Erfindung ist dementsprechend ein Verfahren zur Herstellung von Sclareolid, indem man Sclareol und/oder Abienol in wäßrigem Medium und in Abwesenheit eines organischen Lösungsmittels mit der 10,5- bis 25-fachen molaren Menge - bezogen auf Sclareol und/oder Abienol - eines Oxidationsmittels in Gegenwart eines Ruthenium-Katalysators und eines Emulgators zu einem Rohprodukt umsetzt und dieses entweder
(i) in Gegenwart einer Base in das Salz von 8α-Hydroxy-11-carboxy-12,13,14,15,16-pentanorlabdan überführt und dieses weiter in saurem Medium zu Sclareolid cyclisiert oder
(ii) einer Nachreaktion bei erhöhter Temperatur unterwirft und anschließend destilliert, wobei die Nachreaktion gewünschtenfalls auch im Zuge der Destillation in situ erfolgen kann.

Das erfindungsgemäße Verfahren hat den Vorteil, daß nur ein einziger Oxidationsschritt benötigt wird und Sclareol in hoher Ausbeute zugänglich ist. Darüber hinaus erfolgt die Oxidationsstufe im Vergleich zum zitierten Stand der Technik in Abwesenheit eines organischen Lösungsmittels. Schließlich stellt die Ausführungsform (ii) eine weitere vorteilhafte Form der Verfahrensführung dar, bei der bestimmte Verarbeitungsschritte der Ausführungsform (i) entfallen können.

Das Oxidationsmittel wird in 10,5- bis 25-fachem molarem Überschuß - bezogen auf Sclareol und/oder Abienol - eingesetzt. Ein 12- bis 16-facher Überschuß ist dabei besonders bevorzugt. Als Oxidationsmittel eignen sich z.B. Persäuren, Wasserstoffperoxid oder Alkali- bzw.

Erdalkalihypochlorite. Als besonders günstig hat sich die Verwendung von Oxidationsmittel in Form wäßriger Lösungen erwiesen. Ein besonders gut geeignetes Oxidationsmittel ist Natriumhypochlorit.

Die Art des Emulgators unterliegt an sich keinen besonderen Beschränkungen, jedoch haben sich nichtionische Tenside und insbesondere Anlagerungsprodukte von Ethylen- und/oder Propylenoxid an Fett- bzw. Oxoalkohole als besonders günstig erwiesen. Von besonderem Vorteil ist es dabei, ein Anlagerungsprodukt von 20 Mol Ethylenoxid an handelsüblichen Talgalkohol einzusetzen.

Im Zusammenhang mit einer angestrebten Verkürzung der Reaktionszeit hat es sich herausgestellt, das Reaktionsgemisch, das in Form einer wäßrigen Dispersion vorliegt, vor der eigentlichen Reaktion Bedingungen auszusetzen, die die Ausbildung einer möglichst feinteiligen Dispersion begünstigen. Dazu kommen beispielsweise die Anwendung von Ultraschall, die Verwendung eines Hochgeschwindigkeits-Scherrührwerks oder eines Homogenisators, z.B. vom Typ Supratron oder Cavitron in betracht.

Das Verfahren wird bei Temperaturen von 15 bis 70 °C durchgeführt, vorzugsweise bei 20 bis 50 °C.

Der Ruthenium-Katalysator wird ein einer Menge von 0,1 bis 5 mol-%, vorzugsweise 0,1 bis 1,0 mol-% - bezogen auf Sclareol und/oder Abienol -ein- gesetzt. Es eignet sich sowohl elementares Ruthenium, das entweder in Form eines Pulvers oder auf einem festen Träger, z.B. Aktivkohle oder Aluminiumoxid, eingesetzt werden kann; desweiteren eignen sich Ruthenium-IV-oxidhydrat (RuO₂ x n H₂O) sowie Rutheniumsalze. Als besonders gut geeignet hat sich Rutheniumtrichlorid (RuCl₃) erwiesen.

Das erfindungsgemäße Verfahren wird in der Regel folgendermaßen durchgeführt: Man legt Sclareol und/oder Abienol sowie den Emulgator in Wasser vor, stellt gewünschtenfalls durch Zugabe von Alkalihydroxid alkalisch und gibt den Ruthenium-Katalysator zu. Anschließend dosiert man das Oxidationsmittel zu. Dabei wird ein Rohproduktgemisch erhalten, das überwiegend Sclareolid sowie Nebenprodukte enthält. Die weitere Verarbeitung dieses Rohproduktgemisches erfolgt nach einer der folgenden Varianten:
(i) Die Nebenprodukte werden nach dem weiteren Zusatz einer Base und Erhitzen in das entsprechende Salz der Hydroxysäure (5) überführt; bei diesem Prozeß wird auch Sclareolid durch Ringöffnung in die Hydroxysäure (5) überführt. In einem abschließenden Schritt wird dann die aus dem Salz durch Ansäuern entstandene Hydroxysäure (5) in bekannter Weise durch Wasserabspaltung zu Sclareolid cyclisiert.
(ii) Das bei der Oxidation erhaltene Rohprodukt wird unter speziellen Bedingungen erhitzt, so daß die erwähnten Nebenprodukte ohne zusätzliche Verarbeitungsschritte in Sclareolid umgewandelt werden. Das kann z.B. dadurch geschehen, daß man das Rohprodukt zunächst für ca. 3 Stunden bei Temperaturen um 120 °C erhitzt; anschließend wird dann Sclareolid unter schonenden Bedingungen, beispielsweise mittels eines Dünnschichtverdampfers, abdestilliert. Es kann andererseits in situ dadurch geschehen, daß man das Rohprodukt bei Temperaturen destilliert, die einerseits hoch genug sind, um eine Umwandlung der Nebenprodukte in Sclareolid zu bewirken, und die andererseits schonend genug sind, um eine Zersetzung des Sclareolids weitgehend zu vermeiden. Dies läßt sich etwa durch Destillation bei ca. 140 ° im Hochvakuum erreichen.

Die folgenden Beispiele dienen der Erläuterung der Erfindung und sind nicht einschränkend zu verstehen.

### Beispiele

### 1. Trivialnamen / Abkürzende Bezeichnungen

**Sclareol (1):** 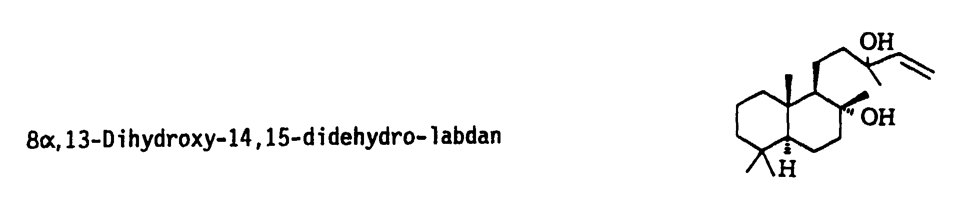
**Abienol (2):** 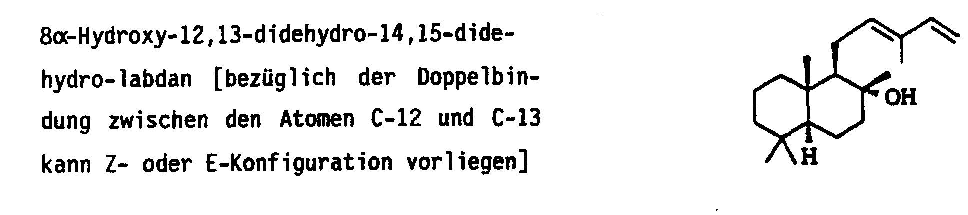
**Hydroxyketon (3):** 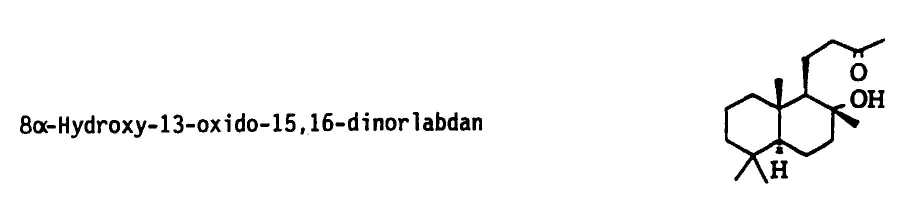
**Enolether (4):** 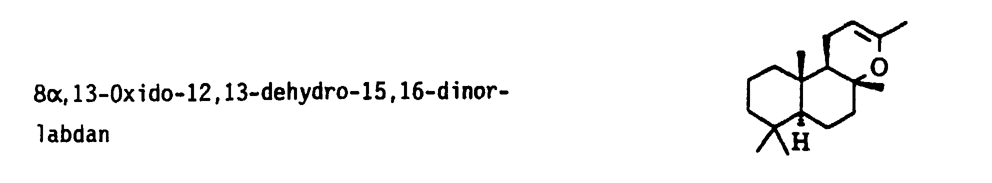
**Hydroxysäure (5):** 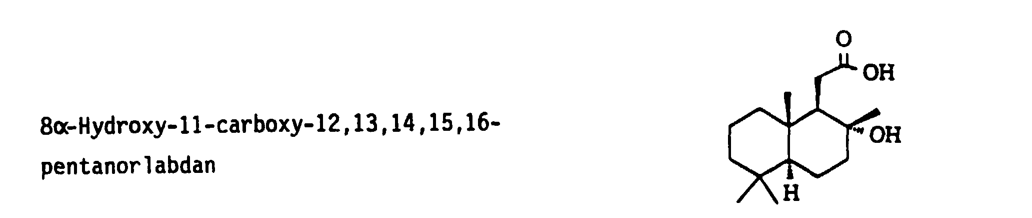
**Sclareolid (6):** 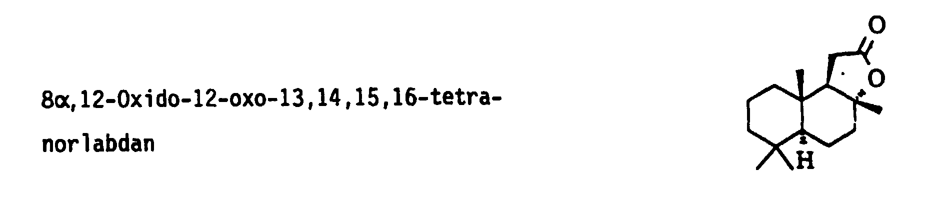
**Ambroxan (7):**

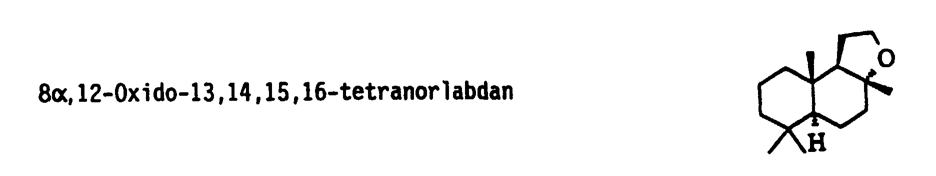

### 2. Herstellbeispiele

Im folgenden sind alle %-Angaben - außer Ausbeuten - als Gew.-% zu verstehen.

Die Beispiele 1 und 2 dienen der Verdeutlichung der Variante (i) [Oxidation von Sclareol zu Roh-Sclareolid, anschließende Überführung der darin enthaltenen Nebenprodukte in die Hydroxysäure und anschließende Cyclisierung der Hydroxysäure zu Sclareolid], wobei Beispiel 2 die Vorteile beim Einsatz einer möglichst feinteiligen Dispersion zeigt.

Beispiel 3 dient der Verdeutlichung der Variante (ii) [Oxidation von Sclareol zu Roh-Sclareolid und dessen Nachreaktion in situ im Zuge der sich anschließenden Destillation].

### Beispiel 1

**(I)** 62 g Sclareol und 1,9 g eines Anlagerungsproduktes von 20 Mol Ethylenoxid an Talgfettalkohol ("Dehydol TA 20"; Fa. Henkel/Düsseldorf) wurden in 350 ml Wasser vorgelegt und unter Rühren auf 80 °C erwärmt. Man ließ die Dispersion auf 35-40 °C abbkühlen, und setzte der Dispersion 0,83 g Rutheniumtrichlorid (25%-ige Lösung; Fa. Degussa) und 112,5 g einer 50%-igen wäßrigen KOH-Lösung zu. Anschließend dosierte man innerhalb von 3 Stunden unter Rühren 1862,5 g einer 13%-igen wäßrigen Natriumhypochloritlösung zu. Nach vollständiger NaOCl-Zugabe wurde über Nacht gerührt, wobei sich der Reaktionsansatz auf Raumtemperatur abkühlte.
**(II)** Zur Aufarbeitung wurde das Gemisch mit 150 ml 40%-iger Schwefelsäure auf einen pH-Wert von 1-2 eingestellt und die wäßrige Phase abgetrennt. Der organische Rückstand wurde in 600 ml Toluol aufgenommen und 2-mal mit 250 ml Wasser gewaschen.
**(III)** Anschließend wurden der organischen Lösung 27,5 g einer 50%-igen NaOH-Lösung und 2,8 g Tatrabutylammoniumchlorid zugegeben und die Reaktionsmischung 5 Stunden bei 60-65 °C gerührt.
   Der Ansatz wurde mit 500 ml Wasser verdünnt, auf 70 °C erhitzt und die organische Phase abgetrennt. Die wäßrige Phase wurde mit 40%-iger Schwefelsäure auf einen pH-Wert von 2 eingestellt und mit 300 ml Toluol extrahiert.
**(IV)** Die toluolische Lösung wurde 4-5 Stunden am Wasserabscheider unter Rückfluß erhitzt. Nach dem Abdestillieren des Lösungsmittels erhielt man Sclareolid in einer Ausbeute von 72,6% der Theorie.

### Beispiel 2

Beispiel 1 wurde wiederholt, wobei als Emulgator 4 g eines Anlagerungsproduktes von 20 Mol Ethylenoxid an Sorbitanmonooleat ("Disponil SMO 120"; Fa. Henkel/Düsseldorf) und 1,5 g Dehydol TA 20 (siehe Beispiel 1) eingesetzt wurden. Die entstandene Dispersion wurde anschließend durch ein Cavitron geführt, wodurch die Reaktionszeit auf 5 Stunden verkürzt wurde. Dabei wurde Sclareolid in einer Ausbeute von 75 % der Theorie erhalten.

### Beispiel 3

Beispiel 2 wurde wiederholt, wobei die Schritte (III) und (IV) ausgelassen wurden. In Schritt (II) wurde stattdessen nach dem Ansäuern und der Phasentrennung der organische Rückstand nicht in Toluol aufgenommen, sondern direkt bei 150 °C im Hochvakuum (0,01 mbar) destilliert. Dabei wurde Sclareolid in einer Ausbeute von 78 % der Theorie erhalten.

## Patentansprüche

1. Verfahren zur Herstellung von Sclareolid, **dadurch gekennzeichnet**, daß man Sclareol und/oder Abienol in wäßrigem Medium und in Abwesenheit eines organischen Lösungsmittels mit der 10,5- bis 25-fachen molaren Menge - bezogen auf Sclareol und/oder Abienol - eines Oxidationsmittels in Gegenwart eines Ruthenium-Katalysators und eines Emulgators zu einem Rohprodukt umsetzt und dieses entweder
(i) in Gegenwart einer Base in das Salz von 8α-Hydroxy-11-carboxy-12,13,14,15,16-pentanorlabdan überführt und dieses in saurem Medium weiter zu Sclareolid cyclisiert oder
(ii) einer Nachreaktion bei erhöhter Temperatur unterwirft und anschließend destilliert, wobei die Nachreaktion gewünschtenfalls auch im Zuge der Destillation in situ erfolgen kann.

2. Verfahren nach Anspruch 1, wobei man als Oxidationsmittel Natriumhypochlorit einsetzt.

3. Verfahren nach Anspruch 1 oder 2, wobei man als Emulgator ein nichtionisches Tensid einsetzt.

4. Verfahren nach einem der Ansprüche 1 bis 3, wobei man die Oxidation bei einer Temperatur von 15 bis 70 °C durchführt.

5. Verfahren nach einem der Ansprüche 1 bis 4, wobei man den Ruthenium-Katalysator in einer Menge von 0,1 bis 5 mol-% - bezogen auf Sclareol und/oder Abienol - einsetzt.

6. Verfahren nach einem der Ansprüche 1 bis 5, wobei man als Ruthenium-katalysator RuCl₃ einsetzt.

7. Verfahren nach einem der Ansprüche 1 bis 6, wobei man die Oxidation in einer möglichst feinteiligen Dispersion durchführt.

## Claims

1. A process for the production of sclareolide, **characterized in that** sclareol and/or abienol is reacted with 10.5 to 25 times the molar quantity - based on sclareol and/or abienol - of an oxidizing agent in aqueous medium in the absence of an organic solvent and in the presence of a ruthenium catalyst and an emulsifier to form a crude product which, thereafter, is either
(i) converted in the presence of a base into the salt of 8α-hydroxy-11-carboxy-12,13,14,15,16-pentanorlabdane which is then cyclized in acidic medium to form sclareolide
or
(ii) is subjected to an after-reaction at elevated temperature and subsequently distilled, the after-reaction optionally taking place in situ during the distillation phase.

2. A process as claimed in claim 1, **characterized in that** sodium hypochlorite is used as the oxidizing agent.

3. A process as claimed in claim 1 or 2, **characterized in that** a nonionic surfactant is used as the emulsifier.

4. A process as claimed in any of claims 1 to 3, **characterized in that** the oxidation is carried out at a temperature of 15 to 70°C.

5. A process as claimed in any of claims 1 to 4, **characterized in that** the ruthenium catalyst is used in a quantity of 0.1 to 5 mole-%, based on sclareol and/or abienol.

6. A process as claimed in any of claims 1 to 5, **characterized in that** RuCl₃ is used as the ruthenium catalyst.

7. A process as claimed in any of claims 1 to 6, **characterized in that** the oxidation is carried out in a particularly fine-particle dispersion.

## Revendications

1. Procédé de préparation de sclaréolide, où on fait réagir le sclaréol et/ou l'abiénol dans un milieu aqueux et en l'absence d'un solvant organique avec une quantité 10, 5 à 25 fois molaires, par rapport au sclaréol et/ou à l'abiénol, d'un oxydant en présence d'un catalyseur au ruthénium et d'un émulsionnant pour donner un produit brut et,
(i) soit on le transforme en présence d'une base en le sel de 8α-hydroxy-11-carboxy-12, 13, 14, 15, 16-pentanorlabdane et on le cyclise en milieu acide en sclaréolide,
(ii) soit on le soumet à une postréaction à température élevée et ensuite on distille, en pouvant réaliser aussi la postréaction si c'est souhaité in situ au cours de la distillation.

2. Procédé selon la revendication 1,
où on utilise comme oxydant de l'hypochlorite de sodium.

3. Procédé selon la revendication 1 ou 2,
où on utilise comme émulsionnant un tensioactif non ionique.

4. Procédé selon l'une des revendications 1 à 3,
où on réalise l'oxydation à une température de 15 à 70°C.

5. Procédé selon l'une des revendications 1 à 4,
où on utilise le catalyseur de ruthénium en une quantité de 0,1 à 5 % molaire, par rapport au sclaréol et/ou à l'abiénol.

6. Procédé selon l'une des revendications 1 à 5,
où on utilise comme catalyseur de ruthénium RuCl₃.

7. Procédé selon l'une des revendications 1 à 6,
où on réalise l'oxydation en une dispersion la plus fine possible.
